**Europäisches Patentamt**

(19) **European Patent Office**

**Office européen des brevets**

(11) Numéro de publication: **0 149 585**

**B1**

(12) **FASCICULE DE BREVET EUROPÉEN**

(45) Date de publication du fascicule du brevet:
14.12.88

(51) Int. Cl.⁴: **C 07 D 301/32,** C 07 D 303/04

(21) Numéro de dépôt: **85420009.4**

(22) Date de dépôt: **16.01.85**

(54) **Procédé d'élimination de gaz dissous dans une solution aquese d'oxyde d'éthylène.**

(30) Priorité: **17.01.84 FR 8400631**

(43) Date de publication de la demande:
**24.07.85 Bulletin 85/30**

(45) Mention de la délivrance du brevet:
**14.12.88 Bulletin 88/50**

(84) Etats contractants désignés:
**BE DE FR GB IT NL SE**

(56) Documents cité:
**US-A-3 766 714**

(73) Titulaire: **ATOCHEM, 4 & 8, Cours Michelet La Défense 10, F-92800 Puteaux (FR)**

(72) Inventeur: **Neel, Henry, 7 Boulevard François Ier, F-76600 Le Havre (FR)**
Inventeur: **Delannoy, Francis, 61 rue Ampère, F-69310 Pierre- Benite (FR)**

## Description

La présente invention concerne le traitement de solutions aqueuses diluées d'oxyde d'éthylène renfermant des gaz dissous pour en éliminer ces gaz.

De telles solutions peuvent avoir diverses origines mais proviennent essentiellement de la synthèse de l'oxyde d'éthylène par oxydation catalytique de l'éthylène en phase gazeuse.

Dans une telle synthèse, l'oxyde d'éthylène formé doit être isolé à partir d'un mélange gazeux le renfermant à l'état très dilué et comprenant entre autres, à côté de l'éthylène et de l'oxygène non transformés, de la vapeur d'eau, du dioxyde de carbone $CO_2$, du méthane, de l'éthane, de l'azote, des gaz rares et d'autres impuretés dont principalement des aldéhydes comme le formaldéhyde et l'acétaldéhyde.

Le processus habituellement adopté pour parvenir à l'oxyde d'éthylène à partir d'un tel mélange gazeux comprend:

a. une absorption à l'eau consistant à mettre en contact le mélange gazeux issu de la zone de réaction avec de l'eau et conduisant à l'obtention d'une solution aqueuse diluée d'oxyde d'éthylène contenant le plus souvent environ 2 à 3 % d'oxyde d'éthylène en poids, des impuretés telles que des aldéhydes, et, à l'état dissous, notamment, du $CO_2$, du méthane, de l'éthane, de l'éthylène, de l'azote, de l'oxygène et des gaz rares.

b. une désorption consistant à soumettre la solution diluée d'oxyde d'éthylène à une extraction à la vapeur d'eau dans une colonne délivrant, en pied, un courant aqueux pratiquement débarassé d'oxyde d'éthylène et, en tête, un mélange gazeux contenant de la vapeur d'eau, des gaz initialement dissous dans la solution aqueuse d'oxyde d'éthylène, des impuretés telles que les aldéhydes et environ 30 à 60 % en poids d'oxyde d'éthylène.

c. une réabsorption par mise en contact du courant gazeux précédent, préalablement refroidi, avec de l'eau. L'oxyde d'éthylène est ainsi réabsorbé dans l'eau ainsi que du $CO_2$ et la majeure partie des impuretés, tandis que la plus grande partie des gaz initialement présents sous forme dissoute avec l'oxyde d'éthylène sont séparés sous forme d'un courant gazeux. La solution aqueuse d'oxyde d'éthylène résultant de l'étape de réabsorption contient généralement entre 5 et 15 % en poids d'oxyde d'éthylène.

d. une ou plusieurs distillations pour obtenir l'oxyde d'éthylène pur à partir de cette dernière solution.

Il est de plus souvent nécessaire d'insérer une étape supplémentaire entre les étapes c et d pour éliminer du $CO_2$. La solution aqueuse d'oxyde d'éthylène ainsi décarbonatée peut être traitée pour faire des glycols, distillée pour obtenir de l'oxyde d'éthylène purifié, ou en partie traitée pour faire des glycols et en partie distillée.

L'invention a pour objet le traitement de solutions aqueuses telles que celles résultant des étapes a et c d'un procédé de synthèse d'oxyde d'éthylène et, plus généralement, celui de toute solution aqueuse diluée d'oxyde d'éthylène renfermant, à l'état dissous, du dioxyde de carbone et au moins un autre composé normalement gazeux et présent à l'état dissous dans les solutions du type cité issues d'un procédé de fabrication d'oxyde d'éthylène, pour en éliminer les gaz dissous, directement à partir de ces solutions, de telle façon qu'après leur séparation, ces gaz puissent être avantageusement recyclés dans la synthèse de l'oxyde d'éthylène sans pratiquement entraîner avec eux de l'oxyde d'éthylène.

En effet si tel n'est pas le cas, il faut procéder à une étape supplémentaire pour séparer de ces gaz l'oxyde d'éthylène qu'ils renferment, avant de pouvoir les recycler à la synthèse de l'oxyde d'éthylène.

Une telle séparation complique évidemment le processus général d'isolement de l'oxyde d'éthylène et n'en favorise pas l'économie.

C'est par exemple un défaut de la technique décrite dans le brevet des Etats-Unis d'Amérique n° 3 729 899 qui consiste à soumettre la solution diluée d'oxyde d'éthylène à une vaporisation éclair. Un autre inconvénient de cette technique est de ne réaliser qu'une séparation partielle, au plus égale à 85 % des gaz initialement dissous dans la solution d'oxyde d'éthylène traitée.

Pour sa part, le brevet des Etats-Unis d'Amérique US-A-3 766 714, obtient une solution aqueuse diluée d'oxyde d'éthylène pratiquement débarrassée de gaz dissous en prévenant la présence de ceux-ci lors même de la formation d'une telle solution par condensation partielle, dans des conditions déterminées, du courant gazeux sortant en tête d'une colonne de désorption telle qu'au point b mentionné plus haut, et en extrayant à contre-courant l'oxyde d'éthylène présent dans la phase gazeuse en équilibre avec la solution diluée d'oxyde d'éthylène lors de la condensation partielle, à l'aide de cette solution même.

Le procédé selon l'invention permet une élimination des gaz dissous qui peut être pratiquement totale tout en ne laissant subsister pratiquement pas d'oxyde d'éthylène dans les gaz séparés de la solution d'oxyde d'éthylène.

Il consiste à mettre en contact une solution aqueuse diluée d'oxyde d'éthylène contenant, à l'état dissous, du dioxyde de carbone et au moins un autre composé normalement gazeux et présent à l'état dissous dans les solutions aqueuses d'oxyde d'éthylène résultant de l'absorption à l'eau de ce produit dans un procédé d'oxydation directe de l'éthylène par l'oxygène moléculaire, avec un courant gazeux constitué d'un ou de plusieurs des gaz suivants azot, éthylène, méthane, éthane et argon et dans une colonne comprenant deux zones comportant chacune jusqu'à 15 plateaux théoriques, la solution aqueuse d'oxyde d'éthylène étant introduite dans la colonne à un niveau situé entre la zone inférieure comprenant au moins 2 plateaux théoriques et la zone supérieure comprenant au moins 5 plateaux théoriques, le courant gazeux étant introduit dans la colonne au-dessous de la zone inférieure et de l'eau étant introduite dans la colonne au-dessus de la zone supérieure, les gaz étant évacués en tête de la colonne et la solution d'oxyde d'éthylène extraite de la base de la colonne après traitement.

Les plateaux théoriques de la colonne peuvent être matérialisés d'une façon connue par des plateaux ou des garnissages réputés pour assurer un contact efficace gaz-liquide.

La pression absolue dans la colonne est avantageusement comprise entre 1 et 20 bars.

Comme déjà indiqué, la solution traitée peut renfermer jusqu'à 15 % en poids d'oxyde d'éthylène. Elle renferme le plus souvent 2 à 12 % en poids d'oxyde d'éthylène. La quantité totale de gaz dissous représente en général moins de 0,5 %, le plus souvent moins de 0,3 % en poids de la solution.

La température de la solution d'oxyde d'éthylène à son entrée dans la colonne est généralement supérieure à la température ambiante et inférieure à 80°C.

L'eau introduite en tête de colonne peut être de l'eau pure ou une eau recyclée dans le procédé oxyde d'éthylène et pouvant contenir en particulier de faibles quantités d'oxyde d'éthylène et/ou de glycol. Ce peut être par exemple une eau analogue à celle assurant l'absorption de l'oxyde d'éthylène à partir des gaz issus de la zone d'oxydation catalytique de l'éthylène. Sa température à son entrée dans la colonne est avantageusement comprise entre 10°C et 50°C. Son débit est égal à 5 à 15 % en poids de celui de la solution d'oxyde d'éthylène.

Le courant gazeux introduit en-dessous de la zone inférieure de la colonne est constitué d'un ou plusieurs gaz choisis parmi l'azote, l'éthylène, le méthane, l'éthane et l'argon. Son débit est généralement compris entre 0,05 % et 10 % en poids du débit de la solution à traiter.

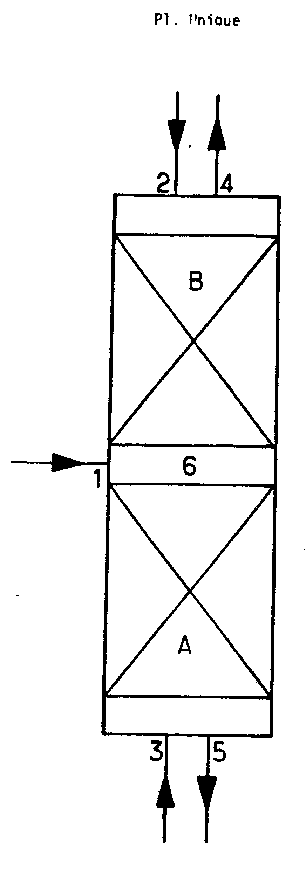

La figure unique présente un schéma du mode de réalisation du procédé selon l'invention.

La colonne 6 comprend deux zones A et B comportant des plateaux réels ou des garnissages équivalents dans chacune d'elles à au plus 15 plateaux théoriques.

La solution aqueuse diluée d'oxyde d'éthylène est introduite en 1 dans la partie de la colonne située entre les zones A et B.

Le courant gazeux servant à l'entraînement des gaz dissous dans la solution d'oxyde d'éthylène est introduit en 3 dans la colonne 6 tandis que l'eau pénètre dans cette même colonne en 2.

En 4 est évacué un courant gazeux essentiellement constitué de gaz initialement dissous dans la solution d'oxyde d'éthylène et du ou des gaz introduits en 3.

En 5 est évacuée la solution d'oxyde d'éthylène traitée.

Les exemples suivants, illustrent le procédé selon l'invention.

**Exemple 1**

Une solution aqueuse d'oxyde d'éthylène renfermant, en poids, 2,55 % d'oxyde d'éthylène, 5,22 % d'éthylèneglycol, 0,17 % de $CO_2$, 0,032 % d'éthylène, 0,015 % d'azote et 0,004 % d'éthane, oxygène et argon globalement, est introduite à raison de 1 902 kg/h et à 72°C, dans la colonne 6 dont les deux zones ont chacune 5 plateaux théoriques.

Le courant gazeux nécessaire à l'entraînement des gaz dissous dans la solution d'oxyde d'éthylène est constitué d'azote introduit dans la colonne 6 au débit de 1,46 kg/h et à 25°C.

Le courant aqueux introduit en tête de colonne, constitué d'eau contenant 5,5 % en poids d'éthylèneglycol, est introduit dans la colonne 6 à raison de 131,23 kg/h, à 25°C.

La pression absolue moyenne dans la colonne 6 est égale à 2,5 bars.

Le courant gazeux évacué en tête de colonne à raison de 5,66 kg/h renferme 99,8 % du $CO_2$ et la totalité des autres gaz initialement dissous dans la solution d'oxyde d'éthylène introduite dans la colonne. Il renferme moins de 0,004 % en poids d'oxyde d'éthylène.

La solution aqueuse d'oxyde d'éthylène évacuée en pied de la colonne au débit de 2 029 kg/h ne renferme plus que 0,003 % en poids de $CO_2$ dissous. Le coefficient d'élimination du $CO_2$ est ainsi de plus de 98 %.

Un résultat analogue est atteint lorsque l'azote est remplacé par la même quantité molaire de méthane.

**Exemple 2**

En opérant comme dans l'exemple 1 mais en utilisant un courant d'éthylène à raison de 1,91 kg/h à la place du courant d'azote, la solution d'oxyde d'éthylène évacuée au bas de la colonne contient seulement 1 % du $CO_2$ initialement dissous dans la solution d'oxyde d'éthylène introduite dans la colonne, à côté seulement de 0,01 % en poids d'éthylène.

Le courant gazeux évacué en tête de la colonne ne contient que 0,005 % en poids d'oxyde d'éthylène.

**Exemple 3**

En opérant comme dans l'exemple 2 mais dans une colonne ne conportant que 2 plateaux théoriques dans la zone inférieure et avec un débit d'éthylène de 3,76 kg/h, le rendement d'élimination du $CO_2$ atteint 99 % tandis que l'élimination des autres gaz initialement dissous dans la solution d'oxyde d'éthylène introduite dans la colonne est complète. Le courant gazeux contenant les gaz éliminés de la solution d'oxyde d'éthylène contient moins de 0,015 % en poids d'oxyde d'éthylène.

## Exemple 4

Une solution aqueuse contenant, en poids, 2,5 % d'oxyde d'éthylène, 0,17 % de $CO_2$ et 0,32 % d'éthylène est introduite à raison de 1 902 kg/h et à 80°C dans la colonne 6 dont les 2 zones ont chacune 5 plateaux théoriques et fonctionnant sous une pression absolue moyenne de 15 bars. 19,4 kg/h d'azote à 25°C et 131 kg/h d'eau à 25°C sont respectivement introduits en-dessous de la zone inférieure et au-dessus de la zone supérieure de la colonne 6.

Le traitement selon l'invention permet d'obtenir une solution d'oxyde d'éthylène ne contenant plus que 1 % du $CO_2$ initial, le rendement d'élimination global des gaz initialement dissous dans la solution à traiter atteignant 94 % et la teneur en oxyde d'éthylène dans le courant gazeux issu en haut de la colonne n'étant que de 0,0005 %.

## Exemple 5

Une solution aqueuse contenant, en poids, 11,73 % d'oxyde d'éthylène, 0,031 % de $CO_2$ et 0,0014 % d'éthylène est introduite, à 42°C, à raison de 1 971 kg/h dans la colonne de l'exemple 4 où elle est mise en contact avec un courant gazeux d'azote introduit dans cette colonne à 25°C à raison de 3,83 kg/h.

De l'eau est introduite en tête de colonne à 25°C au débit de 131 kg/h.

La pression absolue moyenne dans la colonne est de 2,5 bars. La solution d'oxyde d'éthylène extraite de la colonne renferme moins de 1 % de la quantité de $CO_2$ qu'elle contenait initialement, l'élimination des autres gaz dissous étant pratiquement totale.

Le courant gazeux extrait en tête de la colonne ne contient que 0,003 % en poids d'oxyde d'éthylène.

## Revendications

1. Procédé d'élimination des gaz dissous dans une solution aqueuse d'oxyde d'éthylène contenant jusqu'à 15 % en poids d'oxyde d'éthylène et, à l'état dissous, au plus 0,5 % en poids de gaz constitués de dioxyde de carbone et d'au moins un autre composé normalement gazeux et présent à l'état dissous dans les solutions aqueuses d'oxyde d'éthylène résultant de l'absorption à l'eau de ce produit dans un procédé d'oxydation directe de l'éthylène par l'oxygène moléculaire, procédé d'élimination dans lequel la solution aqueuse d'oxyde d'éthylène est mise en contact à contre-courant avec un courant gazeux, caractérisé en ce que le courant gazeux est constitué d'un ou de plusieurs des gaz suivants azote, éthylène, méthane, éthane et argon, et qu'il est mis en contact avec la solution aqueuse d'oxyde d'éthylène dans une colonne comprenant deux zones comportant chacune jusqu'à 15 plateaux théoriques, la solution d'oxyde d'éthylène étant introduite dans la colonne à un niveau situé entre la zone inférieure comprenant au moins 2 plateaux théoriques et la zone supérieure comprenant au moins 5 plateaux théoriques, le courant gazeux étant introduit dans la colonne au-dessous de la zone inférieure, et de l'eau pure ou recylée dans le procédé d'oxyde d'éthylène étant introduite dans la colonne au-dessus de la zone supérieure, les gaz étant évacués en tête de la colonne et la solution d'oxyde d'éthylène extraide sa base après traitement.

2. Procédé selon la revendication 1 caractérisé en ce que le débit du courant gazeux introduit dans la colonne est compris entre 0.05 % et 10 % en poids du débit de la solution aqueuse d'oxyde d'éthylène introduite dans la colonne.

3. Procédé selon l'une quelconque des revendications 1 et 2 caractérisé en ce que la température de la solution aqueuse d'oxyde d'éthylène est comprise entre la température ambiante et 80°C.

4. Procédé selon l'une quelconque des revendications 1 à 3 caractérisé en ce que le débit de l'eau introduite dans la colonne est compris entre 5 et 15 % en poids de celui de la solution aqueuse d'oxyde d'éthylène introduite dans la colonne.

5. Procédé selon l'une quelconque des rvendications 1 à 4 caractérisé en ce que la température de l'eau introduite dans la colonne est comprise entre 10°C et 50°C.

6. Procédé selon l'une quelconque des revendications 1 à 5 caractérisé en ce que l'eau introduite dans la colonne contient de l'éthylèneglycol.

7. Procédé selon l'une quelconque des revendications 1 à 6 caractérisé en ce que la pression absolue moyenne dans la colonne est comprise entre 1 et 20 bars.

## Patentansprüche

1. Verfahren zur Freisetzung von in einer wässrigen Ethylenoxidlösung mit bis zu 15 Gewichtsprozent Ethylenoxid gelösten Gasen, die höchstens 0,5 Gewichtsprozent ausmachen und aus Kohlendioxid und mindestens einer anderen normalerweise gasförmigen Verbindung bestehen und in gelöster Form in den wässrigen Ethylenoxidlösungen enthalten sind, die durch die Aufnahme dieses Produktes durch Wasser in einem Verfahren zur direkten Oxydation von Ethylen durch molekularen Sauerstoff entstehen, Verfahren zur Freisetzung, in dem die wässrige Ethylenoxidlösung im Gegenstrom mit einem Gasstrom in Kontakt gebracht wird, dadurch gekennzeichnet, daß der Gasstrom aus einem oder mehreren der folgenden Gase besteht: Stickstoff, Ethylen, Methan, Ethan und Argon, und daß er mit der wässrigen Ethylenoxidlösung in einer Kolonne in Kontakt gebracht wird, die aus zwei jeweils bis zu 15 theoretische Böden enthaltenden Bereichen besteht, indem die wässrige Ethylenoxidlösung auf einer Höhe zwischen dem mindestens 2 theoretische Böden umfassenden unteren Bereich und dem mindestens 5 theoretische Böden umfassenden oberen Bereich in die Kolonne eingebracht wird, indem der Gasstrom unter dem unteren Bereich in die Kolonne eingebracht wird und indem reines oder im Ethylenoxidverfahren wiederverwendetes Wasser über dem oberen Bereich in die Kolonne eingebracht wird, wobei die Gase am oberen Ende der Kolonne abgeführt werden und die Ethylenoxidlösung an deren unterem Ende nach der Behandlung gewonnen wird.

2. Verfahren nach Patentanspruch 1, dadurch gekennzeichnet, daß die Durchsatzmenge des in die Kolonne eingeführten Gasstroms zwischen 0,05 und 10 Gewichtsprozent der Durchsatzmenge der in die Kolonne eingebrachten wässrigen Ethylenoxidlösung beträgt.

3. Verfahren nach irgendeinem der Patentansprüche 1 und 2, dadurch gekennzeichnet, daß die Temperatur der wässrigen Ethylenoxidlösung zwischen Raumtemperatur und 80°C liegt.

4. Verfahren nach irgendeinem der Patentansprüche 1 bis 3, dadurch gekennzeichnet, daß die Durchsatzmenge des in die Kolonne eingebrachten Wassers zwischen 5 und 15 Gewichtsprozent derjenigen der in die Kolonne eingebrachten wässrigen Ethylenoxidlösung beträgt.

5. Verfahren nach irgendeinem der Patentansprüche 1 bis 4, dadurch gekennzeichnet, daß die Temperatur des in die Kolonne eingeführten Wassers zwischen 10° und 50°C liegt.

6. Verfahren nach irgendeinem der Patentansrüche 1 bis 5, dadurch gekennzeichnet, daß das in die Kolonne eingeführte Wasser Ethylenglykol enthält.

7. Verfahren nach irgendeinem der Patentansprüche 1 bis 6, dadurch gekennzeichnet, daß der mittlere Absolutdruck in der Kolonne zwischen 1 und 20 bar liegt.

**Claims**

1. A process for removing gases dissolved in an aqueous solution of ethylene oxide containing up to 15 % ethylene oxide by weight, at most 0.5 % dissolved carbon dioxide by weight, and at least one other normally gaseous compound present in the dissolved state in aqueous solutions of ethylene oxide resulting from absorption of this product in water during a process of direct oxidation of ethylene by molecular oxygen, this removal process being one in which the aqueous solution of ethylene oxide is brought into counter-current contact with a gaseous stream, characterized in that the gaseous stream consists of one or more of the following gases: nitrogen, ethylene, methane, ethane and argon, and that it is brought into contact with the aqueous ethylene oxide solution in a column comprising two zones each comprising up to 15 theoretical plates, the aqueous ethylene oxide solution being introduced into the column at a level between the lower zone comprising at least 2 theoretical plates and the upper zone comprising at least 5 theoretical plates, the gaseous stream being introduced into the column below the lower zone and pure water or water recycled in the ethylene oxide process being introduced into the column above the upper zone, the gases being removed from the top of the column and the ethylene oxide solution being removed from its base after treatment.

2. A process according to Claim 1, characterized in that the gaseous stream introduced into the column is supplied at a rate between 0.05 % and 10 % by weight of the rate of supply of aqueous ethylene oxide solution introduced into the column.

3. A process according to any one of Claims 1 and 2 characterized in that the temperature of the aqueous ethylene oxide solution is between ambient temperature and 80°C.

4. A process according to any one of Claims 1 to 3 characterized in that water introduced into the column is supplied at a rate between 5 and 15 % by weight of that of the aqueous ethylene oxide solution introduced into the column.

5. A process according to any one of Claims 1 to 4 characterized in that the temperature of the water introduced into the column is between 10°C and 50°C.

6. A process according to any one of Claims 1 to 5 characterized in that the water introduced into the column contains ethylene glycol.

7. A process according to any one of Claims 1 to 6 characterized in that the average absolute pressure in the column is between 1 and 20 bars.

Pl. Unique